# EUROPEAN PATENT SPECIFICATION

(11) **EP 0 326 925 B1**
(45) Date of publication and mention of the grant of the patent: **10.05.1995**
(21) Application number: 89101267.6
(22) Date of filing: 25.01.1989
(51) Int. Cl.: E04F 13/08, E04C 2/00

(54) **Anchor board system**
Ankersystem für Platten
Système d'ancrage pour panneaux

(30) Priority: 26.01.1988 US 148711
(43) Date of publication of application: 09.08.1989
(73) Proprietor: TAC-FAST Systems SA, CH-1752 Villars-sur-Glâne (CH)
(72) Inventor: Pacione, Joseph Rocco, Thornhill, Ontario (CA)
(74) Representative: VOSSIUS & PARTNER

(56) References cited:
- DE-A- 2 744 009
- DE-U- 7 029 524
- DE-U- 7 706 130
- US-A- 3 499 102

## Description

This invention is directed to a construction system embodying an anchor plaster board for use in walls and like surfaces, and providing an outer anchor surface to which finish surface units can be removably adhered in press-fitting hooked attachment thereto.

In the construction and interior decorating industry, widespread use is made of drywall construction wherein interior area enclosures such as interior surface walls and partition walls are constructed having a plasterboard or like finished member secured thereto. The plasterboard is generally secured in place in nailed attachment to wooden or sheet metal framing members and subsequently finished. The finishing of the exterior surfaces of the drywall construction generally requires a large amount of skilled hand labor, and involves the application of plaster over adjoining board joints, the application of a joint tape and the subsequent application of two or more layers of plaster to conceal the joint. The process, in addition to requiring skilled labor is time consuming and generates quantities of fine chalk dust as a consequence of the necessary intervening sanding-down procedures, which dust of itself constitutes a health hazard, as well as being a general nuisance. This type of drywall construction generally entails the attachment and secondary finishing of door, window and other trim.

Thus, once the prepatory work is completed, requisite door, cove and other trim is applied and the painters can commence the application of subcoats and finishing coats of paint; the other trades such as tilers can apply tile, and decorative paneling can be glued or nailed into place. The labor-intensive nature of all the necessary stages is self-evident, and well known.

DE-U-70 29 524 teaches the application of attachment strips attached, as an intermediate carrier, to a surface such as a wall or a ceiling, in order that covering material may be applied over the surface.

In order to overcome the above drawbacks, the present invention provides an anchor plaster board according to claim 1, a system for forming and finishing interior surfaces of a building according to claim 12, and a finishing method according to claim 18.

An anchor plaster board suitable for forming the interior wall of a building as a replacement for drywall by in-place cutting, fitting and attaching such board to the interior structural members of the building comprises a board which is integrally manufactured with one half of a hook and loop attachment system projecting from and covering substantially the entire surface of one face of the board and which is capable of being trimmed as required for fitting to form the interior wall of a building by attachment in place to the structural members.

In a further aspect of the invention, the anchor plaster board cooperates with surface finishing units having complementary hook or loop means on their back.

The subject hook means generally are distributed over substantially one full surface of the board unit, i.e. its anterior face.

The present invention in addition in another aspect provides a system including components, such as anchor boards, for detachable application to a surface of a support strata in semi-permanent relation by way of fastening means comprising a hook and loop combination, the component having a reverse surface thereof incorporating over at least a portion of the area thereof a plurality of fastening elements selected from the hooks and loops for removably securing the component to the support strata.

The support strata can form a part of a support member such as a beam or truss having an obverse face area incorporating a complementary one of the fastening means to receive the component in removable adherent relation thereto. Such a double-faced horizontal I-joist can support two facings of anchor board, as a partition.

The component, in turn, generally comprises an anchor board of extensive area, the obverse face thereof incorporating over at least a portion of the area thereof, a plurality of one of the fastening elements, to receive a further component in removable adherent relation thereto.

The system in one preferred embodiment comprises a wall system supported by an accessible surface of a support member. The component comprising an anchor board is detachably secured to the support member surface in covering relation therewith.

Further system components attachable in removable adhering relation to the anchor board are selected singly or in combination from the group comprising surface finish units, trim sections, including cove and baseboard, and utility enclosures.

Edge portions of the board unit may be pre-profiled to facilitate forming of external corners, wherein a diminution of board thickness can accommodate the application of suitable corner trim in substantially flush fitting relation.

Further, it is contemplated that specialty anchor boards, having one edge border of thickened proportions, may be provided where a stylized thin baseboard of deep section may be applied thereon.

Ancillary boards may also be provided to supplement the board thickness, for decorative and other purposes, such as utility services, for supporting wiring conduits or other services in concealed, externally accessible relation, for the location of electrical wiring, etc. for power, communication, computer and other services.

This aspect of the invention has the great advantage of permitting wiring to be carried out substantially completely subsequent to the finishing of a room, thereby eliminating preliminary rough wiring. This affords a particular advantage that internal arrangements, such as location of desks and any associated electrical equipment, such as computers, printers etc. can be totally flexible, in relation to adjacent wall surfaces.

The construction of the subject boards generally is based upon plasterboard units having high fire retardent characteristics. In the matter of fire retardency, the use of hook means comprising a hook formation of substantially flameproof, non-toxic plastic material is contemplated, so as to form an anchor strata.

In one embodiment, the hook means is secured and protrudes from a substantially continuous film, whereby in manufacturing the anchor board the thus formed continuous film or other anchor strata may be readily incorporated into the board manufacturing process. The incorporation into the anchor strata of protruding root members, or perforations for setting into the plaster, or the provision of a surface active adherent layer to effect adherence of the hook strata to the plaster material of the board body is contemplated, together with utilization of a paperboard outersheet layer as the reverse board face.

While one embodiment incorporates the subject anchor layer into a plaster board formulation, it will be understood that incorporation of the anchor strata layer into other board formulation embodiments also lies within the ambit of the present invention. Normally, the board will be manufactured with the hook or loop attachment system incorporated in or on the board so that the board can be transported to a site and cut and fit in place like conventional drywall.

A further embodiment comprises an anchor board having an attachment hook strata on the obverse face, as above described, and an attachment loop strata on the reverse board face, for use in cooperation with structural supporting members appropriately equipped with hook or loop outer surfaces, to receive the anchor board in adhering attachment thereto. A double-faced I-section and an adaptor flange for attachment to a two-by-four inch (5.08 by 10.16cm) joist, each having hook or loop elements on their surface areas, are cases in point.

Characteristic board dimensions generally comprise rectangular boards in standard two foot (61 cm) and four foot (122 cm) widths, in standard one foot (30.5 cm) increments in length from six feet (183 cm) to ten feet (305 cm). Other sizes may well be adopted in accordance with user needs.

Board thicknesses comprise adopted standards, generally a nominal one-half inch (12.7 mm) thickness or three-quarter inch (19.05 mm) thickness, excluding the height of the anchor strata. Board thickness may vary, on the adoption of different materials and in conformity with building codes and other requirements. It will be appreciated that the nature of the resultant engineered structure ultimately provided will be changed, owing to the application of a "dress surface" to the anchor strata, to provide a composite structure having a resilient intermediate anchor strata. Benefits in sound absorbency characteristics and some improvement in the currently negligible thermal insulation "R" values are anticipated.

Handling of the subject boards in pairs, having the anchor strata faces in adjoining face to face relation is contemplated, with probable weight restrictions on vertical stacking, to avoid hook damage.

Attachment of the anchor boards to floors, walls and ceilings is contemplated generally by nailing, stapling, gluing and the like. However, the extension of the system, using respective hook and loop strata, for attachment of the anchor boards to underlying supports is considered part of the system, which thus includes the underlying supports with their respective loop or hook attachment strata.

It will be appreciated that the anchor strata construction can incorporate some reinforcement to the boards, thus facilitating the use of stapling machines to wire staple the boards to studding and other strata. The anchor boards, being a more precisely engineered product than existing wall board, with associated savings in cost for the completed system, may economically enable the use of fasteners of a type that would pull through existing paper faced plasterboard, to thereby enable further reductions in installation labor costs by the adoption of more cost effective fastening techniques. Owing to the use of an anterior cover layer with the subject anchor board, there are few limitations on the nature of the anchor board fastening means that can be adopted. In the case of the above referred to support system for supporting the anchor boards, it is possible to substantially dispense with other fastening means such as screws and nails, and rely upon the disclosed loop and hook formulations.

Certain embodiments of the present invention are described, reference being made to the accompanying drawings, wherein:
Figure 1 is a general view of a typical anchor board in accordance with the present invention;
Figure 2 is an enlarged general view of the portion 2 of Figure 1;
Figure 3 is a general view of a portion of a partition wall incorporating wall elements in accordance with the inventions;
Figure 4 is an edge section of a portion of a cover unit for use in combination with the subject anchor boards;
Figure 5 is an enlarged perspective view of a conduit forming a further component of the subject systems, in attached relation with a further anchor board embodiment;
Figure 6 is a vertical section of partition wall and associated floor incorporating elements of the subject system in accordance with the present invention;
Figure 7 is a vertical section of certain alternative system elements in accordance with the present invention;
Figure 8 is a plan view of an anchor board corner assembly and corner trim;
Figure 9 is a perspective view of a portion of an I-beam joist by which a partition is suspended; and
Figure 10 is a perspective scrap view of a partition joist having a system flange plate attached thereto.

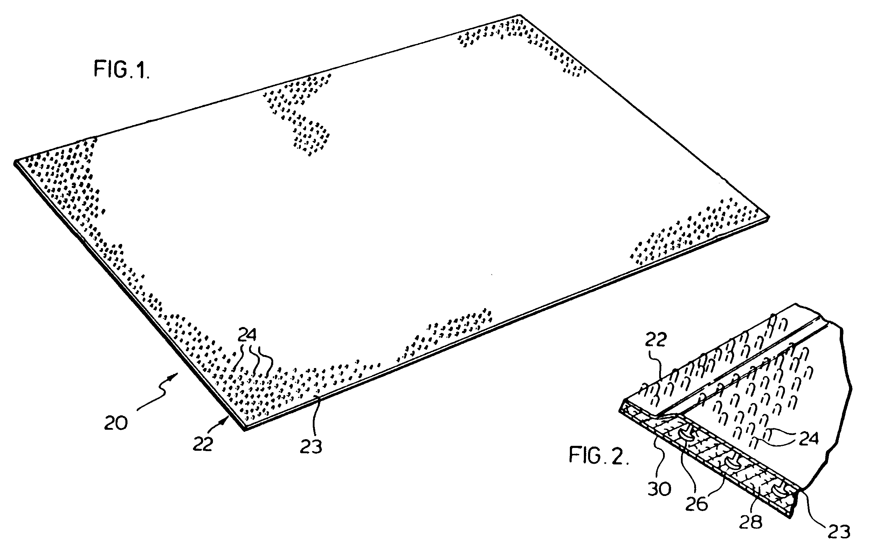

Referring to Figure 1, this shows an anchor board 20 having typical dimensions of a four feet (122 cm) width by eight feet (244 cm) length, a base thickness of one half inch, i.e., 12.7 mm(nominal) and an upper strata 22 (see also Figure 2), the full area of which incorporates a strata sheet 23, having embedded hook portions 24, and rearwardly extended strata retention portions 26. The body portion 28 of the illustrated embodiment comprises plaster, bounded by a posterior paperboard layer 30.

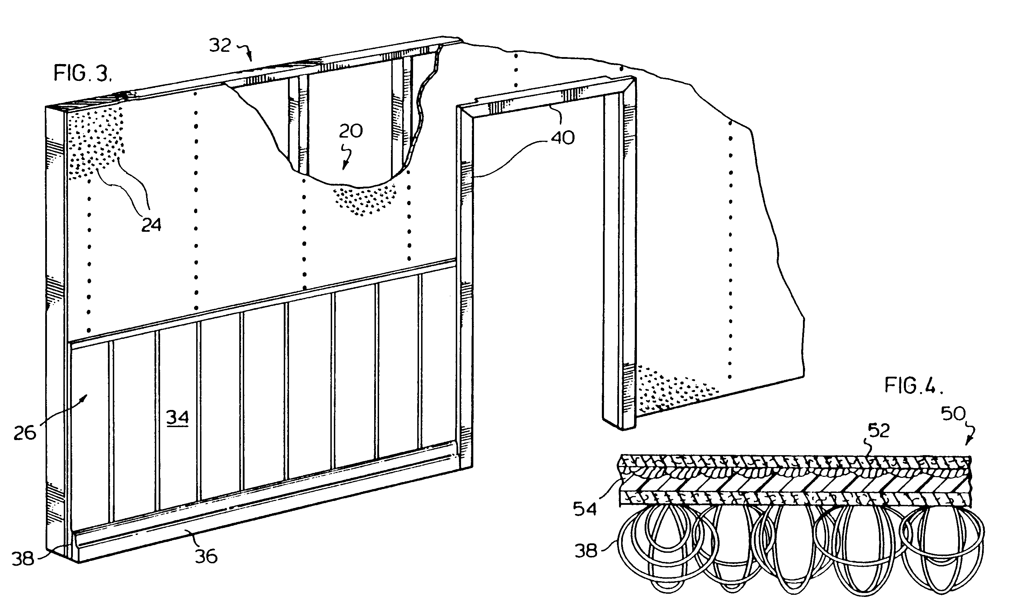

Referring to the system embodiment illustrated in Figure 3, a wall frame 32 of conventional two by four foot (61 cm by 122 cm) wood construction or of prefabricated sheet metal joists has anchor sheeting 20 nailed thereto, being illustrated as having a wainscotting sheeting 34 affixed thereto by way of the anchoring hook portions 24, which covers substantially the whole of the wall surface 26.

A skirting board 36 is adhered by way of hook portions 24 with a looped while 38 forming a backing to the skirting board 36. A doorway is bounded by trim molding 40, also adhered to the anchor sheeting 20 byway of looped pile (not shown). The door molding 40 may have a recessed peripheral rear edge, to accommodate an overlay of the edge of wainscotting sheets 34 in securing and concealing relation therewith.

Referring to Figure 4, a portion of a finish sheet 50 is shown, in enlarged relation, having a finished anterior surface 52, an intermediate laminate or glue portion 54 and an adherent layer 38 of woven or felted loops, for securement to the hook portions 24 of an underlying anchor sheet 20.

The term "underlying" relates the anchor sheet 20 to the overlying anterior surface 52, which could well be that of a ceiling tile, or decorative wallboard and the like, or could be a fabric covering or carpet tile or even a plastic paper covering.

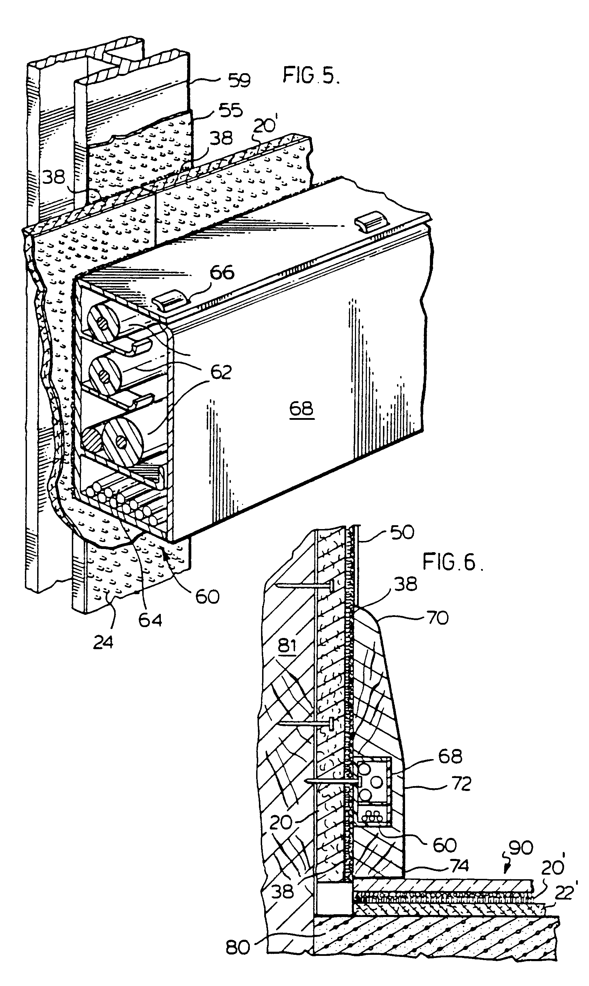

Figure 5 shows a portion of a conduit tray 60, illustrated as being of shallow depth, with accommodation for three or more electrical conductors 62 and a separate compartment 64 to receive telephone and other telecommunications lines and computer lines therein.

Hinge apertures 66 receive attachment lugs of a flame-proof cover plate 68. The anchor board 20′ of Figure 5, in addition to a hooked anterior face, with hook portions 24 also has the posterior face thereof with looped pile 38 on at least edge portions of board 20′, to secure the board 20′ to the illustrated hooked surface 55 of structural member 59.

Referring to Figure 6, the cove 70 has a downwardly extending lip 72 which conceals and retains the conduit coverplate 68. A primary or filler baseboard 74 is adhered to the anchor sheet 20 and serves to support conduit tray 60.

In Figure 6 it is shown how the anchor sheets 20′, probably having a particle board base portion 22′ can be installed on a subfloor 80. An anchor sheet 20 of the above-disclosed plaster or other suitable base is secured to stud 81, in nailed or other secured relation. The conduit 60 is shown as being also screw-nailed into place, in addition to the subject anchor hooks. A carpet 90, also secured in similar fashion to anchor sheet 20' is covered at its edge by primary base board 74, which serves as an edge trim for the carpet 90.

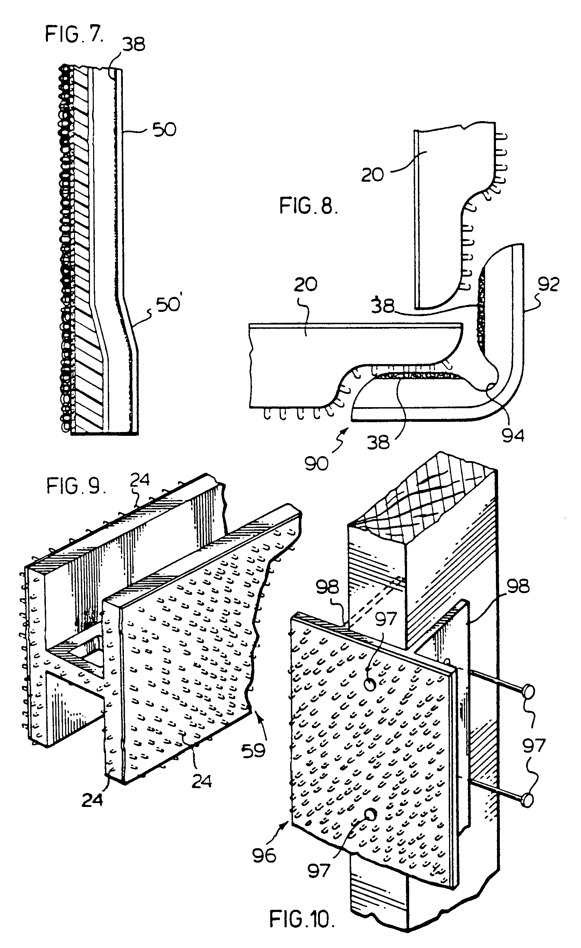

Figure 9 shows an I-section joist 59 having system hook elements 24 on the externally accessible surfaces, and service access ways provided through the web of the joist 59. In Figure 10 is shown an attachable flange 96 secured by nails 97 and side pads 98 to a two-by-four inch (5.08 by 10.16 cm) joist, for attachment of an anchor board to the thus enlarged hooked flange surface.

It will be appreciated that the anchor sheet and the combination fitting for use therewith lend themselves to mass production, with associated cost savings. The respective fittings, with the anchor sheet, combine into a system which lends itself to extremely rapid erection, tear-down and/or replacement. Furthermore, the accessory component parts are substantially undamaged by installation and removal from the subject anchor sheets. The present system provides enhanced utility to both commercial and private property, with the capability to refinish a room or hallway, office and the like with great rapidity and low labor charges. Re-utilization of related combination parts makes leasing of interior wall decor a distinct possibility.

Furthermore, the associated flexibility, including the provision of services in concealed accessible relation with minimal labor costs, makes the replanning and reorganization of office accommodations much less time consuming and minimizes business disruption, while reducing labor costs.

Referring to Figure 8, it can be seen how the provision of diminished board edge borders facilitates the achievement of a neat external corner whereby a trim piece 90 having a desired external finish 92, a felted or looped posterior surface 38, and a substantially flexible bight 94 permits the provision of a substantially flush external corner, to which the adjacent finished surfaces can be precisely butted.

While the subject anchor board is disclosed and illustrated primarily as having a hooked surface attachment means to receive finish surface units in attached relation thereto, it will be appreciated that the fastening means is an element of a hook and loop combination. Thus, one or other of the fastening elements may be adopted for a specific surface, and the complementary element adopted for the respective complementary attachable component of the system.

## Claims

1. An anchor plaster board suitable for forming the interior wall of a building as a replacement for drywall by in-place cutting, fitting and attaching such board to the interior structural members (59) of the building, characterized in that the board (20) is manufactured with an attachment system integral with the plaster board, with one half of a hook and loop attachment system (24, 38) projecting from and covering substantially the entire surface of one face of the board so as to be capable of being trimmed as required for fitting, to form the interior wall of a building by attachment in place to the structural members.

2. The anchor plaster board of claim 1 in which the one half of the hook and loop attachment system is embedded into the plaster board (20).

3. The anchor plaster board of claim 2 in which the one half of the hook and loop attachment system (24, 38) is connected to a support strata (26) embedded into the plaster board.

4. The anchor plaster board of claim 3 in which the one half of the hook and loop attachment system (24, 38) is hooks.

5. The anchor plaster board of claim 4 in which the hooks are made of flame-retardent, non-toxic plastic material.

6. The anchor plaster board of claim 5 in which the hooks project from the anterior surface.

7. In combination with the anchor plaster board of any of claims 1 to 6, a finish unit having a complementary half of a hook and loop attachment system (24, 38) on a posterior surface removably adherable to the anterior surface of the anchor plaster board (20).

8. The combination of claim 7 in which the finish unit has on one surface loops (38), and the board (20) has complementary hooks (24) on one surface for attachment of the finish unit.

9. The combination of claim 8 in which the finish unit has, on its surface opposite the loops, a finish selected from either paper, plastic cloth or wood.

10. The combination of claim 9 in further combination with wiring conduit means having loops (38) on one surface for attachment to the complementary hooks on the board (20).

11. In combination with the anchor plaster board of any of claims 1 to 6, a finish unit having complementary loops (38) on one surface, a finished surface of paper, cloth or wood on an opposite surface so that such finish unit is removably adherable to the board (20).

12. A system, for forming and finishing interior surfaces of a building, including one part of a hook and loop attachment system (24, 38) and the finishing material (34) having a complementary part of the hook and loop attachment system (24, 38) on a reverse face thereof, characterized in that the system includes substantially rigid panels (20) manufactured with the attachment system integral with each of the panels, with one half of the hook and loop attachment system (24, 38) projecting from a front major face to cover at least substantially over the whole of the front face such that the panels are capable of being cut to the shape required, attached to a wall framework (59) and have the finishing material (34) attached to the front faces of the panels.

13. The system of claim 12, in which the panels (20) additionally have one half of a hook and loop attachment system projecting (24, 38) from a rear major face adapted to attach to another half of a hook and loop system provided on the wall framework (59).

14. The system of claim 12, in which the panels are of plasterboard construction.

15. The system of claim 12, wherein said one half of the hook and loop attachment system projecting from the front major faces of the panels comprises hooks (24).

16. The system of claim 12, in which the finishing material (34) comprises a cover sheet to cover joins between panels.

17. The system of claim 16, in which the cover sheet is selected from the group consisting of wall paper, textiles, plastics and paneling.

18. A method of finishing an interior wall of a building using an anchor plaster board of any of claims 1 to 6, comprising the steps of:
(a) transporting a plurality of the anchor plaster board panels to a location to be finished;
(b) trimming the panels to fit edge to edge with one another and with boundaries of the location to be finished to form interior surfaces;
(c) attaching the panels at the location to be finished with said one face to the interior thereof;
(d) adding surface finish sheet having a complementary one-half of a hook and loop system.

19. The method as claimed in claim 18, in which the support is a wall framework and the panels are of plasterboard construction.

20. The method as claimed in claim 19, in which the finish sheet is selected from wallpaper, textiles, plastic and paneling.

21. The method as claimed in claim 18, in which the support is a floor and the panels are selected from the group consisting of plywood and particle board.

22. The method as claimed in claim 21, in which the finish sheet is carpet.

23. The method as claimed in claim 18, in which each of the panels contains hooks and the finish sheet contains loops.

24. The method as claimed in claim 18, in which the finish sheet hides joints between adjoining panels.

## Patentansprüche

1. Verankerungs-Gipsbauplatte, die zum Ausbilden der Innenwand eines Gebäudes als ein Trockenwandersatz geeignet ist, indem eine derartige Platte vor Ort zugeschnitten, eingepaßt und an den Innenstrukturelementen (59) des Gebäudes befestigt wird, dadurch gekennzeichnet, daß die Platte (20) mit einem Befestigungssystem in einstückiger Form mit der Gipsbauplatte gefertigt ist, wobei eine Hälfte eines Haken/Schlingen-Befestigungssystems (24, 38) im wesentlichen die gesamte Oberfläche einer Seite der Platte bedeckt und aus dieser hervorsteht, so daß sie nach Bedarf zum Einpassen zugerichtet werden kann, um die Innenwand eines Gebäudes durch Befestigung an den Strukturelementen zu bilden.

2. Verankerungs-Gipsbauplatte nach Anspruch 1, bei welcher die eine Hälfte des Haken/Schlingen-Befestigungssystems in der Gipsbauplatte (20) eingebettet ist.

3. Verankerungs-Gipsbauplatte nach Anspruch 2, bei welcher die eine Hälfte des Haken/Schlingen-Befestigungssystems (24, 38) mit einer in der Gipsbauplatte eingebetteten Stützschicht (26) verbunden ist.

4. Verankerungs-Gipsbauplatte nach Anspruch 3, bei welcher die eine Hälfte des Haken/Schlingen-Befestigungssystems (24, 38) aus Haken besteht.

5. Verankerungs-Gipsbauplatte nach Anspruch 4, bei welcher die Haken aus flammenhemmendem, nicht toxischem Kunststoffmaterial bestehen.

6. Verankerungs-Gipsbauplatte nach Anspruch 5, bei welcher die Haken aus der Vorderseite hervorstehen.

7. Fertigoberflächeneinheit mit einer komplementären Hälfte eines Haken/Schlingen-Befestigungssystems (24, 38) auf einer Rückseite, die auf der Vorderseite der Verankerungs-Gipsbauplatte (20) entfernbar befestigbar ist, in Kombination mit der Verankerungs-Gipsbauplatte nach einem der Ansprüche 1 bis 6.

8. Kombination nach Anspruch 7, bei welcher die Fertigoberflächeneinheit auf der einen Oberfläche Schlingen (38), und die Platte (20) komplementäre Haken (24) auf einer Oberfläche zur Befestigung der Fertigoberflächeneinheit aufweist.

9. Kombination nach Anspruch 8, bei welcher die Fertigoberflächeneinheit an ihrer den Schlingen gegenüberliegenden Oberfläche eine aus Papier, Kunststofftuch oder Holz ausgewählte Fertigoberfläche aufweist.

10. Kombination nach Anspruch 9 in weiterer Kombination mit einer Kabelkanaleinrichtung, welche Schlingen (38) auf der einen Oberfläche zur Befestigung an den komplementären Haken auf der Platte (20) aufweist.

11. Fertigoberflächeneinheit, welche komplementäre Schlingen (38) auf einer Oberfläche und eine Fertigoberfläche aus Papier, Stoff oder Holz an einer gegenüberliegenden Oberfläche aufweist, so daß eine derartige Fertigoberflächeneinheit entfernbar an der Platte (20) befestigbar ist, in Kombination mit der Verankerungs-Gipsbauplatte nach einem der Ansprüche 1 bis 6.

12. System zum Ausbilden und Endbearbeiten von Innenflächen eines Gebäudes, welches einen Teil eines Haken/Schlingen-Befestigungssystems (24, 38) aufweist und wobei das Fertigoberflächenmaterial (34) einen komplementären Teil des Haken/Schlingen-Befestigungssystems (24, 38) auf seiner Rückseite aufweist, dadurch gekennzeichnet, daß das System im wesentlichen starre Paneele (20) enthält, die jeweils einstückig mit dem Befestigungssystem gefertigt sind, wobei eine Hälfte des Haken/Schlingen-Befestigungssystems (24, 38) aus einer Hauptvorderseite hervorsteht, um zumindest im wesentlichen die gesamte Vorderseite derart zu Überdecken, daß die Paneele auf die erforderliche Form zugeschnitten, an einem Wandrahmentragwerk (59) befestigt worden können, und das Fertigoberflächenmaterial (34) an den Vorderseiten der Paneele befestigt werden kann.

13. System nach Anspruch 12, bei welchem zusätzlich eine Hälfte eines Haken/Schlingen-Befestigungssystems (24, 38) aus einer hinteren Hauptfläche der Paneele(20) hervorsteht, welches angepaßt ist, an einer anderen an dem Wandrahmentragwerk (59) vorgesehenen Hälfte eines Haken/Schlingen-Befestigungssystems anzuhaften.

14. System nach Anspruch 12, bei welchem die Paneele den Aufbau einer Gipsbauplatte aufweisen.

15. System nach Anspruch 12, wobei die eine Hälfte des Haken/Schlingen-Befestigungssystems, die aus den vorderen Hauptflächen der Paneele hervorsteht, Haken (24) aufweist.

16. System nach Anspruch 12, bei welchem das Fertigoberflächenmaterial (34) eine Deckschicht aufweist, um Verbindungsstellen zwischen Paneelen zu überdecken.

17. System nach Anspruch 16, bei welchem die Deckschicht aus der aus Tapeten, Textilien, Kunststoffen und Täfelung bestehenden Gruppe ausgewählt wird.

18. Verfahren zum Ausführen der Endoberfläche einer Innenwand eines Gebäudes unter Verwendung einer Verankerungs-Gipsbauplatte nach einem der Ansprüche 1 bis 6; mit den Schritten:
(a) Transportieren mehrerer Verankerungs-Gipsbauplatten zu einer mit der Endoberfläche zu versehenden Stelle;
(b) Zurichten der Platten, um die Kanten auf Stoß zueinander und zu den Begrenzungen der mit der Endoberfläche zu versehenden Stelle anzupassen, um Innenoberflächen herzustellen;
(c) Befestigen der Platten an der mit der Endoberfläche zu versehenden Stelle mit der einen Seite nach innen;
(d) Hinzufügen einer Fertigoberflächenschicht, welche eine komplementäre Einzelhälfte eines Haken/Schlingen-Systems aufweist.

19. Verfahren nach Anspruch 18, bei welchem die Unterlage ein Wandrahmentragwerk ist und die Platten die Konstruktion einer Gipsbauplatte aufweisen.

20. Verfahren nach Anspruch 19, bei welchem die Fertigoberflächenschicht aus Tapeten, Textilien, Kunststoffen und Täfelung ausgewählt wird.

21. Verfahren nach Anspruch 18, bei welchem die Unterlage ein Boden ist und die Platten aus der aus Sperrholz und Spanplatten bestehenden Gruppe ausgewählt werden.

22. Verfahren nach Anspruch 21, bei welchem die Fertigoberfläche Teppich ist.

23. Verfahren nach Anspruch 18, bei welchem jede Platte Haken und die Fertigoberfläche Schlingen enthält.

24. Verfahren nach Anspruch 18, bei welchem die Fertigoberfläche Verbindungsstellen zwischen aneinanderstoßenden Platten verbirgt.

## Revendications

1. Plaque de plâtre accrochable appropriée pour la réalisation d'un mur d'intérieur d'un bâtiment en remplacement d'un mur en matériau sec, en coupant, ajustant et accrochant sur place des éléments (59) de structure intérieurs du bâtiment, caractérisée en ce que la plaque (20) est réalisée avec un système d'accrochage intégré à ladite plaque de plâtre, une moitié d'un système (24, 38) d'accrochage par crochets et boucles faisant saillie à partir d'une des faces de ladite plaque et couvrant sensiblement toute la surface de celle-ci, de façon à pouvoir être appliquée de la façon requise, pour former le mur d'intérieur d'un bâtiment par accrochage en place sur les éléments de structure.

2. Plaque de plâtre accrochable selon la revendication 1, dans laquelle l'une des moitiés du système d'accrochage par crochets et boucles est prise dans la plaque de plâtre (20).

3. Plaque de plâtre accrochable selon la revendication 2, dans laquelle l'une des moitiés du système d'accrochage par crochets et boucles (24, 38), est liée à une couche de support (26) prise dans la plaque de plâtre.

4. Plaque de plâtre accrochable selon la revendication 3, dans laquelle la moitié du système d'accrochage (24, 38) par crochets et boucles est constituée de crochets.

5. Plaque de plâtre accrochable selon la revendication 4, dans laquelle les crochets sont constitués de matière plastique ignifuge et non toxique.

6. Plaque de plâtre accrochable selon la revendication 5, dans laquelle les crochets font saillie à partir de la surface antérieure.

7. En combinaison avec la plaque de plâtre de l'une quelconque des revendications 1 à 6, une unité de finition, présentant, sur une surface postérieure, une moitié complémentaire d'un système d'accrochage (24, 38) par crochets et boucles, capable d'adhérer de façon amovible a la surface antérieure de la plaque de plâtre (20) accrochable.

8. Combinaison selon la revendication 7, dans laquelle l'unité de finition comporte sur une surface des boucles (38) et la plaque de plâtre (20) comporte les crochets complémentaires (24) sur une surface, à des fins d'accrochage sur l'unité de finition.

9. Combinaison selon la revendication 8, dans laquelle l'unité de finition comporte sur sa surface opposée aux boucles une couche de revêtement choisie parmi le papier, la toile plastique et le bois.

10. Combinaison selon la revendication 9, comprenant de plus des moyens de guidage de fils comportant des crochets (38) sur une surface à des fins d'accrochage aux crochets complémentaires de la plaque (20).

11. Unite de finition, en combinaison avec la plaque de plâtre accrochable selon l'une quelconque des revendications 1 à 6, comportant des crochets (38) complémentaires sur une surface, une face de finition constituée de papier, de toile ou de bois et sur une face opposée, de façon que l'unité de finition puisse adhérer de manière amovible à la plaque de plâtre (20).

12. Système pour former des surfaces intérieures d'un bâtiment et en à réaliser les finitions, incluant une partie d'un système d'accrochage (24, 38) par crochets et boucles, un élément de finition (34) comportant une partie complémentaire du système d'accrochage (24, 38) par crochets et boucles sur une face opposée de celui-ci, caractérisé en ce que le système comprend des panneaux (20) sensiblement rigides, le système d'accrochage étant intégré à chacun des panneaux, une moitié du système d'accrochage (24, 38) par crochets et boucles faisant saillie à partir d'une face principale avant, et couvrant au moins substantiellement la totalité de la face frontale de manière à permettre le découpage des panneaux selon le schéma requis, leur fixation sur une structure murale (59) et l'accrochage de l'élément de finition (34) soit fixé sur les faces frontales des panneaux.

13. Système selon la revendication 12, dans lequel les panneaux (20) présentent en outre une moitié d'une système d'accrochage (24, 38) par crochets et boucles faisant saillie à partir d'une surface principale arrière, adaptée pour se fixer sur une autre moitié d'un système d'accrochage à crochets et à boucles, prévu sur la structure murale (59).

14. Système selon la revendication 12, dans lequel les panneaux sont constitués par des plaques de plâtre.

15. Système selon la revendication 12, dans lequel ladite moitié du système d'accrochage à crochets et à boucles faisant saillie à partir des faces principales avant des panneaux est constituée de crochets (24).

16. Système selon la revendication 12, dans lequel le matériau de finition (34) comprend une couche de revêtement destinée à couvrir les joints existant entre les panneaux.

17. Système selon la revendication 16, dans lequel la couche de revêtement est choisie parmi le papier, les textiles, les plastiques et le lambris.

18. Procédé de finition d'une paroi intérieure d'un bâtiment à l'aide d'une plaque de plâtre accrochable selon l'une quelconque des revendications 1 à 6, comprenant les étapes consistant à :
(a) transporter une pluralité de panneaux formant plaques accrochables vers un site devant recevoir des finitions ;
(b) appliquer les panneaux, de façon à les adapter bord à bord les uns avec les autres et avec les limites du site recevant les finitions, pour former les surfaces intérieures ;
(c) fixer les panneaux sur le site à achever, ladite face étant orientée vers l'intérieur de celui-ci ;
(d) ajouter la couche de finition de surface comportant une moitié complémentaire d'un système d'accrochage à crochets et à boucles.

19. Procédé selon la revendication 18, dans lequel le support est une structure murale et les panneaux sont constitués par des plaques de plâtre.

20. Procédé selon la revendication 19, dans lequel la couche de finition est choisie parmi du papier mural, des textiles, des plastiques et du lambris.

21. Procédé tel que revendiqué dans la revendication 18, dans lequel le support est un plancher et les panneaux sont choisis parmi le groupe comprenant les plaques de contreplaqué et les panneaux de particules.

22. Procédé tel que revendiqué dans la revendication 21, dans lequel la couche de finition est du tapis.

23. Procédé tel que revendiqué dans la revendication 18, dans lequel chacun des panneaux comporte des crochets et la couche de finition comporte des boucles.

24. Procédé tel que revendiqué dans la revendication 18, dans lequel la couche de finition dissimule les joints entre panneaux adjacents.
